Europäisches Patentamt

European Patent Office

Office européen des brevets

(11) Publication number: **0 422 599 A1**

# EUROPEAN PATENT APPLICATION

(21) Application number: 90119373.0

(51) Int. Cl.⁵: **G01N 33/546**, G01N 33/569

(22) Date of filing: **10.10.90**

The applicant has filed a statement in accordance with Rule 28 (4) EPC (issue of a sample only to an expert). Accession number(s) of the deposit(s): IFO 14073.

(30) Priority: **12.10.89 JP 266003/89**
**30.07.90 JP 202267/90**

(43) Date of publication of application:
**17.04.91 Bulletin 91/16**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Yamamoto, Eiji**
**2245-23, Miwa, Yamato-cho**
**Kumage-gun, Yamaguchi 743-01(JP)**
Inventor: **Mizuta, Shigeru**
**2000-33,Shinjo-shimookihara**
**Yanai, Yamaguchi 742(JP)**
Inventor: **Kobayashi, Takashi**
**8-32, Mitsui 3-chome**
**Hikari, Yamaguchi 743(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) **Antigen-sensitized bead and use thereof.**

(57) Synthetic resin beads sesitized with antigens after the treatment with aldehydes have good stability. When said beads are used as the solid phase in enzyme-linked immunosorbent assay, the assay precision, detection sensitivity and reproducibility of antibody titer assay can be unexpectely improved with reduced non-specific reactions.

EP 0 422 599 A1

## ANTIGEN-SENSITIZED BEAD AND USE THEREOF

FIELD OF THE INVENTION

The present invention relates to antigen-sensitized beads and use thereof. More particularly, the present invention relates to synthetic resin beads sensitized with an antigen such as, for example, a Bordetella pertussis -derived antigen, to a method for producing the same and to a method for assaying an antibody against such antigen

BACKGROUND OF THE INVENTION

The pertussis vaccines used in the past were whole-cell vaccines derived from strains of Bordetella pertussis . Frequent occurrence, in 1974, of accidents due to vaccination caused suspension of mass vaccination with pertussis vaccines. With this as a driving force, pertussis vaccines became a matter of concern and interest, whereby studies of Bordetella pertussis and improvement of pertussis vaccines were accelerated.

In 1981, a purified precipitated pertussis vaccine mainly composed of filamentous hemagglutinin (FHA) and pertussis toxin (PT), both considered to be the main components derivable from Bordetella pertussis and effective in protection against infection, was developed and it is now in public use for vaccination. It is therefore very important and desirable to assay antibodies against FHA and PT, as a means for the efficacy evaluation of vaccines and further for the diagnosis and prognostic management of pertussal diseases.

Enzyme-linked immunosorbent assay (ELISA) using microplates is a method so far known for determining the titer of antibodies to FHA and PT [Sato et al., Seminars in Infectious Diseases,4, 380 (1982)]. It has problems, however, from the viewpoints of detection sensitivity, assay precision, reproducibility, nonspecific reactions, reagent stability and so forth. Accordingly, the advent of an improved method and/or reagents for antibody titer determination has been waited for.

SUMMARY OF THE INVENTION

It is an object of the invention to improve, in assaying antibodies against antigens, more particularly antibodies against Bordetella pertussis -derived antigens (hereinafter also referred to as "pertussis antibodies"), the assay precision, detection sensitivity and reproducibility and reduce nonspecific reactions. It is another object of the invention to provide reagents for assay for antibodies such as, for example, pertussis antibody which have good stability.

The present inventors made intensive investigations to establish a method which can assay, especially pertussis antibodies in an accurate and precise manner and in a simple and easy manner even in clinical practice and, as a result, unexpectedly found that synthetic resin beads sensitized with a Bordetella pertussis -derived antigen after treatment with aldehydes have good stability and that when said beads are used as the solid phase in enzyme-linked immunosorbent assay, the assay precision, detection sensitivity and reproducibility of the pertussis antibody titer assay can be improved with reduced nonspecific reactions. Further investigations based on these findings have now led to completion of the present invention.

Thus the invention provides:

(1) synthetic resin beads sensitized with an antigen, which is treated with a compound selected from aldehydes prior to sensitization;

(2) a method of producing the beads mentioned above in (1) which comprises sensitizing a synthetic resin bead with an antigen after the treatment of a compound selected from aldehydes; and

(3) a method of assaying an antibody to an antigen in a liquid sample in the manner of enzyme immunoassay, which method comprises using the beads mentioned above in (1) as a solid phase.

DETAILED DESCRIPTION OF THE INVENTION

The antigens which can be used according to the present invention include, for example, antigens derived from pathogenic micro-organisms such as Bordetella pertussis, Clostridium tetani , Corynebacterium diphtheriae, measles virus, rubella virus, mumps virus, and Japanese encephalitis virus, more particularly antigen selected from active components of the protective antigen constituent of vaccines.

The antigen used in the present invention is preferably an antigen derived from pathogenic bacteria such as Bordetella pertussis , Clostridium tetani , and Corynebacterium diphtheriae and most preferably an antigen derived from Bordetella pertussis (Especially, FHA and PT). As Bordetella pertussis strains usable in the practice wherein the antigen derived from Bordetella pertussis is used as a sensitizing antigen, there may be mentioned, among others, Bordetella pertussis Phase I strains such as B. pertussis Tohama Phase I strain (IFO 14073), B. pertussis Yamaguchi Phase I strain and B.pertussis Phase I 18-323 strain. As the Bordetella pertussis -derived antigen, there may be mentioned, for instance, filamentous hemagglutinin (FHA), pertussis toxin (PT), heat labile toxin, Fimbriae, adenylate cyclase, endotoxin, agglutinogen and the like [Igaku Saikingaku (Medical Bacteriology), volume 1, page 63, 1986 (Saikon Shuppan)] as well as 69K OMP (69 KDa outer membrane protein) and the like [Infection and Immunity, 56 , 3189 (1988)]. Bordetella pertussis Phase I strains, in particular Bordetella pertussis Tohama Phase I strain, are preferred among Bordetella pertussis strains, and FHA or PT 15 is preferred as the Bordetella pertussis -derived antigen.

As the synthetic resin beads to be used as the supporting phase for giving the desired solid phase (sensitized solid phase), there may be mentioned spheroidal, preferably spherical, synthetic resin beads having a diameter of about 0.1 $\mu$m $\sim$ 10 mm, preferably about 2 to 9 mm, more preferably about 3 to 8 mm. It is preferable that the beads have a roughened surface resulting from abrasion.

The synthetic resin bead may also be selected from those whose surface is made of synthetic resin and moreover whose inside is made of, for example, a magnetic substance and the like.

The synthetic resin may be a thermoplastic resin, such as polyethylene, polyamide or polystyrene, or a thermosetting resin, such as urea resin, melamine resin or phenolic resin. The use of a styrene resin, in particular a synthetic resin mainly composed of polystyrene, is preferred, however.

The antigen-sensitized beads according to the invention may be produced by sensitizing the synthetic resin beads mentioned above with an antigen such as a Bordetella pertussis -derived antigen using the known method [Eiji Ishikawa (author): Koso Men-eki Sokuteiho (Enzyme Immunoassay) (Igaku Shoin); Immunoenzymatic Assay Techniques, p.165, 1980 (Martinus Nijhoff); and elsewhere]. It is, however, advantageous to subject the synthetic resin beads to washing as pretreatment prior to sensitization with the antigen since contaminant proteins and other impurities can be removed by such washing and the detection sensitivity in assay for antibodies such as pertussis antibody can be improved thereby.

In a preferred mode of washing treatment, use is made of solutions containing about 0.1 to 5% (v/v), preferably about 1 to 2% (v/v), of a nonionic surfactant, such as a polyoxyethylenesorbitan fatty acid ester (e.g. polyoxyethylenesorbitan monooleate, polyoxyethylenesorbitan monostearate, polyoxyethylenesorbitan monopalmitate, polyoxyethylenesorbitan monolaurate), a polyoxyethylene-sorbitol fatty acid ester (e.g. polyoxyethylenesorbitol monolaurate), a polyoxyethylene fatty acid ester (e.g. polyoxyethylene stearate), a polyoxyethylene higher alcohol ether (e.g. polyoxyethylene-lauryl alcohol, polyoxyethylene-oleyl alcohol), a polyoxyethylene alkylaryl ether (e.g. polyoxyethylene-nonylphenol), a polyoxyethylene-castor oilderivative (e.g. a polyoxyethylene-hardened castor oil derivative such as HCO-50 or HCO-60), a polyoxyethylene-lanolin derivative, a polyoxyethylene-lanolin alcohol derivative or a block polymer type nonionic surfactant (e.g. Pluronic, L-62, L-64, F-68), preferably a polyoxyethylenesorbitan fatty acid ester, more preferably polyoxyethylenesorbitan monolaurate (Tween 20), and washing is carried out at about 10 ° to 50° C, preferably about 20 ° to 30° C, for about 1 to 10 hours, preferably about 2 to 3 hours, once to about 10 times, preferably about 3 to 5 times.

For pretreatment prior to sensitization with the antigen, the synthetic resin bead is advantageously treated with an aldehyde (e.g. formaldehyde, acetaldehyde, pyruvic aldehyde, glutaraldehyde, glyoxal), preferably glutaraldehyde, formaldehyde or acetaldehyde, more preferably glutaraldehyde, for improved detection sensitivity in assay for antibody such as pertussis antibody and for improved stability of the assay reagent for antibodies such as pertussis antibody (synthetic resin beads sensitized with the antigen, for example, Bordetella pertussis -derived antigen).

For aldehyde treatment, an aqueous solution of an aldehyde, preferably glutaraldehyde, is used in a concentration of about 0.1 to 12.5% (v/v), preferably about 0.8 to 2.5% (v/v), and the synthetic resin beads are treated therewith at about 10 ° to 50° C, preferably about 20° to 30° C, for about 10 minutes to 5 hours, preferably about 1 to 2 hours.

A combination of the above-mentioned washing treatment and aldehyde treatment is preferred as the pretreatment prior to sensitization with the antigen and it is preferable to subject the synthetic resin beads to washing treatment, then drying them as necessary, and treat them with an aldehyde. The synthetic resin beads (preferably after the above-mentioned pretreatment) should preferably be washed, prior to sensitization, with the same buffer solution as is used for the antigen preparation to be mentioned herein below

3

When the Bordetella pertussis -derived antigen is used as the antigen according to the present invention, it can be obtained, for example, by purifying from the Bordetella pertussis Tohama Phase I strain culture supernatant by a per se known method [Infection and Immunity, 41 , 313 (1983)]. The Bordetella pertussis -derived antigen thus obtained is generally dissolved in a weekly alkaline buffer solution (preferably with a pH of about 9 to 10) to a concentration of about 0.5 to 5 μg/ml, preferably about 0.8 to 1.5 μg/ml, and the resulting solution is used for sensization with the antigen. Preferred as the buffer solution is a carbonate buffer solution with a pH of about 9.6, the carbonate buffer concentration being about 0.005 to 0.05 M, preferably about 0.0125 to 0.025 M. The antigen solution prepared by dissolving the antigen, for example, the Bordetella pertussis -derived antigen in a buffer solution as described above may be used for antigen sensitization either as it is or after a stabilization treatment which comprises allowing the solution to stand at about 10 ° to 40 °C, preferably about 20 ° to 30 °C, for about 1 to 10 hours, preferably about 4 to 6 hours, more preferably about 2 to 3 hours, prior to sensitization with the antigen. Such stabilization treatment can reduce the data dispersion in assay for antibodies, for example, pertussis antibody and improve the precision of the assay.

After the above-mentioned respective pretreatments as desired, the synthetic resin beads and the antigen such as Bordetella pertussis -derived antigen are subjected to treatment for sensitization with the antigen. For example, the synthetic resin beads are added to the antigen solution and the mixture is allowed to stand overnight at about 4°C, whereby antigen-sensitized beads, namely the synthetic resin beads sensitized with the antigen such as Bordetella pertussis -derived antigen, can be produced.

The antigen-sensitized beads thus obtained can be used, after washing (e.g. with phosphate buffer solution containing 0.1% (v/v) of Tween 20) as necessary, for antibody assay such as, for example, pertussis antibody assay. It is preferable, however, to perform a blocking procedure to reduce nonspecific reactions in the assay, if necessary followed by washing (e.g. with a phosphate buffer solution containing 0.1% (v/v) of Tween 20).

In the blocking procedure, an animal serum (e.g. goat, sheep, bovine, horse, rabbit, guinea pig, mouse or human serum), preferably a goat or sheep serum, is used, together with a buffer solution, for example a phosphate buffer solution containing 0.1% (v/v) of Tween 20. The serum is added to a concentration of about 1 to 20% (v/v), preferably about 5 to 10% (v/v), and the blocking treatment is carried out at a temperature of about 2 ° to 60 °C, preferable about 45 °C, for a period of about 1 to 30 hours, preferably about 2 to 20 hours. In cases where the serum or plasma of an animal is used as the liquid sample to be mentioned hereinbelow, the serum of an animal different in species from the animal mentioned above is usedin the blocking procedure.

The antigen-sensitized beads according to the invention are used as the solid phase (sensitized solid phase) in enzyme-linked immunosorbent assay of liquid samples (e.g. sera or plasmas of animals (human, mouse, rabbit, guinea pig, sheep, goat, horse, cattle, etc.) for pertussis antibodies. Thus, for example, the rabbit, guinea pig, sheep, goat, horse, cattle, etc.) for antibodies (e.g. pertussis antibody). For example, the pertussis antibody titer of a liquid sample (about 10 μ l of human serum or the like) can be determined by reacting said sample generally with about 2 to 4 antigen-sensitized beads of the above kind, further adding an enzyme-labeled antibody capable of reacting with a pertussis antibody (immunoglobulin), and determining the enzyme activity of the resulting immobilized enzyme lable.

The antigen-sensitized beads to be used in assaying antibodies against pathogenic micro-organisms (e.g. pertussis antibody etc.) may be synthetic resin beads sensitized with one antigen derived from the pathogenic micro-organisms (e.g. Bordetella pertussis -derived antibody etc.) or may include two or more synthetic resin bead species respectively sensitized with two or more antigens derived from the pathogenic micro-organisms (e.g. PT and FHA derived from Bordetella pertussis ), for example synthetic resin beads sensitized with PT and synthetic resin beads sensitized with FHA. In the latter case, two or more antibodies (e.g. anti-PT antibody and anti-FHA antibody) in one liquid sample can be assayed simultaniously by placing the two or more bead species in the same container.

The synthetic resin beads sensitized with an antigen such as a Bordetella pertussis -derived antigen as providedby the present invention are stable and quite suited for use as reagents for assaying antibodies against, for example, Bordetella pertussis -derived antigen etc. By using said beads in such assaying, the precision, detection sensitivity and reproducibility of the assay can be improved with reduced nonspecific reactions. Therefore, said beads can be used advantageously in assaying antibodies, for example, pertussis antibodies, etc. for the diagnosis or prognostic management of diseases, for example, pertussis diseases or for the efficacy evaluation of vaccines, for example, pertussis vaccines.

The following examples illustrate the invention in further detail. It goes without saying that they are given only for illustrative purposes and are by no means limitative of the scope of the invention

The characteristics of the Bordetella pertussis Tohama Phase I strain used in the following examples are described, for example, in Infection and Immunity, 6 , 899 (1972). Said strain has been maintained at the National Institute of Health, Tokyo, Japan (NIHJ) and deposited with the Institute for Fermentation, Osaka, Japan under the accession number IFO 14073 since August 13, 1980.

Example 1 (Preparation of PT and FHA)

The pertussis-derived antigens PT and FHA were prepared essentially by the method of Sato et al. [e.g. Igaku Saikingaku (Medical Bacteriology), volume 1, page 62, 1986 (Saikon Shuppan); Infection and Immunity, 1 , 313 (1983)] and 3 mg (in 93 ml) of PT and 7 mg (in 36 ml) of FHA were obtained from 10 liters of a culture fluid.

Thus, the Bordetella pertussis Tohama Phase I strain (IFO 14073) was inoculated into modified Steiner-Scholdt fluid medium and cultured at 35°C for days. The culture supernatant obtained was centrifuged (8,000 rpm, 30 minutes) and the culture supernatant thus obtained was applied to a hydroxyapatite column (pH 8.0) and separated into an effluent fraction (fraction 1) and a fraction (fraction 2) eluated with 0.1 M phosphate buffer (pH 7.0) containing 0.5 M NaCl. The fraction 1 was adjusted to pH 6.0 with sodium hydroxide and hydrochloric acid and applied to a hydroxyapatite column (pH 6.0), a fraction eluted with 0.1 M phosphate buffer (pH 7.0) containing 0. 5 M NaCl was applied to a haptoglobin column, and a fraction eluted with 3 M KSCN was dialyzed against 50% glycerin dissolved in 0.1 M phosphate buffer (pH 7.0) containing 0.5 M NaCl to give purified PT. On the other hand, the fraction 2 was applied to a haptoglobin column, the effluent fraction was subjected to sucrose density gradient centifugation (30,000 rpm, 18 hours, 1 to 26% (v/v) sucrose concentration fractions) and the thus-obtained FHA fraction was dialyzed against 50% glycerin dissolved in 0.1 M phosphate buffer (pH 7.0) containing 0.5 M NaCl to give purified FHA.

Example 1-1

(Pretreatment of polystyrene beads)

A 1% (v/v) aqueous solution (200 ml) of Tween (Funakoshi Yakuhin) was added to 1,000 polystyrene beads (6.35 mm in diameter; Immunochemical Co., Okayama, Japan) and, after treatment at room temperature for 2 hours (five treatments in total), washed with purified water and dried. Then, 200 ml of glutaraldehyde (GA) solution was added to these 1,000 polystyrene beads to a final concentration of 2.5% (v/v) and, after treatment at room temperature for 3 hours, the beads were washed with M/60 carbonate buffer (pH 9.6).

Example 1-2

(Pretreatment of the antigens)

The PT and FHA prepared in Example 1 were pretreated with carbonate buffer by dissolving the PT and FHA antigens in M/60 carbonate buffer (pH 9.6) to concentrations of PT = 1.5 $\mu$g/ml and FHA = 1.0 $\mu$g/ml and allowing the solutions to stand at room temperature for 3 hours.

Example 1-3

(Preparation of a PT-bound solid phase)

The PT antigen solution (160 ml) prepared and treated in Example 1-2 was added to the 1,000 polystyrene beads pretreated in Example 1-1. The mixture was stirred, then degassed under vacuum for 2 hours, allowed to stand overnight at 2-8 °C, and washed with phosphate buffer (PBS) containing 0.1% (v/v) of Tween 20.

Example 1-4

(Preparation of a FHA-bound solid phase)

The FHA antigen solution (160 ml) prepared and treated in Example 1-2 was added to the 1,000 polystyrene beads pretreated in Example 1-1. The mixture was stirred, then degassed under vacuum for 2 hours and, after overnight standing at 2-8° C, washed with 0.1% Tween 20/PBS.

(Blocking)

To the antigen-sensitized solid phase (1,000 beads) obtained in Example 1-3 or 1-4 was added 200 ml of 0.1% Tween 20/PBS containing 10% (v/v) of sheep serum

The mixture was treated on warm water at 45° C for 2 hours with gentle shaking, then washed with 0.1% Tween 20/PBS, and stored in 0.1% Tween 20/PBS containing 10% sheep serum.

Example 1-6

(Assay with the antigen-sesitized solid phase)

(Method)

A buffer solution (0.1% Tween 20/PBS-1 0% sheep serum) was distributed in 1,200-$\mu$l portions into the wells of reaction plated (24-well Falcon Multiwell), followed by addition of 10- $\mu$l portions of a human serum sample or a standard solution (one of serial doubling dilutions). To each well was added to one of the PT- or FHA-bound balls obtained in Example 1-5. The first reaction was carried out at 37° C for 60 minutes in a thermostatic bath. After washing with 0.1% Tween 20/PBS (three times in all), a peroxidase-labeled anti-human IgG antibody (1,200 $\mu$l) was added, and the second reaction was carried out at room temperature for 30 minutes. After completion of the reaction and washing with 0.1% Tween/PBS, the beads were respectively transferred to Clinicon tubes. To each tube was added 500 $\mu$l of a substrate solution (citrate buffer solution containing 0.2 mg/ml o-phenylenediamine and 0.02% $H_2O_2$, pH 4.9), and the reaction was allowed to proceed at room temperature for 30 minutes and then stopped by addition of 1,500 $\mu$l of 1 N $H_2SO_4$, followed by absorbance measurement at 492 nm.

(Result)

(1) Effect of pretreatment of polystyrene beads with glutaraldehyde (GA)

Antigen-sensitized solid phases were produced as described in Example 1-1 to Example 1-5 while varying the final concentration of GA in Example 1-1 within the range of 0 to 2.5% and were used for assaying as described in Example 1-6. The assay sensitivity increases approximately with the increase in GA concentration until an approximately constant level was attained (cf. Table 1). Separately, antigen-sensitized solid phases prepared in the same manner while varying the final GA concentration as described above were stored at 45° C for 5 days and then used for assay purposes as described in Example 1-6. The assay results obtained were comparable to those obtained by using the antigen-sensitized solid phases prepared in Example 1-5 directly after their preparation. The accelerated stability test (45° C, 5 days) thus revealed that the antigen-sensitized solid phases according to the invention are excellent in stability. It is further found that treatment of polystyrene beads with GA prior to sensitization with the antigens can result in more improvements in the stability of the antigen-sensitized solid phases than no treatment with GA.

EP 0 422 599 A1

Table 1

| Sensitizing antigen | Final GA concentration (%) | Standard a-PT | | | | |
|---|---|---|---|---|---|---|
| | | 230EU/ml | 46EU/ml | 9EU/ml | 2EU/ml | 0EU/ml |
| PT | 25 | 1092 | 428 | 125 | 42 | 22 |
| | 12.5 | 1004 | 472 | 126 | 47 | 27 |
| | 6.3 | 1043 | 469 | 131 | 47 | 29 |
| | 3.1 | 917 | 463 | 122 | 43 | 28 |
| | 1.6 | 910 | 391 | 112 | 33 | 19 |
| | 0.8 | 916 | 414 | 109 | 34 | 18 |
| | 0 | 644 | 273 | 62 | 16 | 6 |
| Sensitizing antigen | Final GA concentration (%) | Standard a-FHA | | | | |
| | | 124EU/ml | 25EU/ml | 5EU/ml | 1EU/ml | 0EU/ml |
| FHA | 25 | 796 | 302 | 85 | 28 | 13 |
| | 12.5 | 870 | 376 | 108 | 35 | 16 |
| | 6.3 | 956 | 415 | 114 | 37 | 16 |
| | 3.1 | 852 | 378 | 106 | 35 | 15 |
| | 1.6 | 850 | 351 | 102 | 32 | 18 |
| | 0.8 | 797 | 335 | 92 | 31 | 16 |
| | 0 | 709 | 280 | 73 | 32 | 21 |
| Data given in terms of absorbance (x 1000) | | | | | | |

Example 2

Polystyrene beads were treated according to the same manner as described in Example 1 except that 2.5% (v/v) aqueous solution of acetaldehyde (AA) in place of glutaraldehyde was used. The antigen-sensitized solid phase thus obtained was used in the assay.

Example 2-1

(Pretreatment of polystyrene beads)

A 1% (v/v) aqueous solution (200 ml) of Tween (Funakoshi Yakuhin) was added to 1,000 polystyrene beads (6.35 mm in diameter; Immunochemichal Co., Okayama, Japan) and, after treatment at room temperature for 2 hours (five treatments in total), washed with purified water and dried. Then, 100 ml of acetaldehyde (AA) solution was added to these 1,000 polystyrene beads to a final concentration of 2.5% (v/v) and, after treatment at room temperature for 3 hours, the beads were washed with M/60 carbonate buffer (pH 9.6).

7

Example 2-2

(Pretreatment of the antigens)

The FHA prepared in Example 1 were pretreated with carbonate buffer by dissolving the FHA antigens in M/60 carbonate buffer (pH 9.6) to concentrations of FHA = 1.0 $\mu$g/ml and allowing the solutions to stand at room temperature for 3 hours.

Example 2-3

(Preparation of an FHA-bound solid phase)

The FHA antigen solution (160 ml) prepared and treated in Example 2-2 was added to the 1,000 polystyrene beads pretreated in Example 2-1. The mixture was stirred, then degassed under vacuum for 2 hours and, after overnight standing at 2-8 °C, washed with 0.1 % Tween 20/PBS.

Example 2-4

(Blocking)

To the antigen-sensitized solid phase (1,000 beads each) obtained in Example 2-3 was added 200 ml of 0.1% Tween 20/PBS containing 10% (v/v) of sheep serum. The mixture was treated on warm water at 45 °C for 2 hours with gentle shaking, then washed with 0.1% Tween 20/PBS, and stored in 0.1% Tween 20/PBS containing 10% sheep serum.

Example 2-5

(Assay with the antigen-sensitized solid phases)

(Method)

A buffer solution (0.1% Tween 20/PBS-10% sheep serum) was distributed in 1,200-$\mu$l portions into the wells of reaction plates (24-well Falcon Multiwell), followed by addition of 10-$\mu$l portions of a human serum sample or a standard solution (one of serial doubling dilutions). To each well was added one of the FHA-bound balls obtained in Example 2-5. The first reaction was carried out at 37 °C for 60 minutes in a thermostatic bath. After washing with 0.1% Tween 20/PBS (three times in all), a peroxidase-labeled anti-human IgG antibody (1,200 $\mu$l) was added, and the second reaction was carried out at room temperature for 30 minutes. After completion of the reaction and washing with 0.1% Tween/PBS, the beads were respectively transferred to Clinicon tubes. To each tube was added 500 $\mu$l of a substrate solution (citrate buffer solution containing 0.2 mg/ml o-phenylenediamine and 0.02% $H_2O_2$, pH 4.9), and the reaction was allowed to proceed at room temperature for 30 minutes and then stopped by addition of 1,500 $\mu$l of 1 N $H_2SO_4$, followed by absorbance measurement at 492 nm.

(Results)

(1) Effect of pretreatment of polystyrene beads with acetaldehyde (AA)

Antigen-sensitized solid phases were produced as described in Example 2-1 to Example 2-4 wherein AA was added to a final concentration of 2.5% and were used for assaying as described in Example 2-5. It was found that the assay sensitivity increases more than that in no AA treatment (cf.Table 2).

Table 2

| Sensitizing antigen | Final AA concentration (%) | Standard a-FHA | | | | | |
|---|---|---|---|---|---|---|---|
| | | 200 EU/ml | 50 EU/ml | 12.5 EU/ml | 3.12 EU/ml | 0.78 EU/ml | 0 EU/ml |
| FHA | 2.5 | 826 | 338 | 113 | 40 | 23 | 17 |
| | 0 | 720 | 304 | 107 | 37 | 21 | 13 |
| Data given in terms of absorbance (x 1000) | | | | | | | |

Example 3

Antigen-sensitized solid phases were prepared according to the same procedures as described in Example except that various antigen listed in Table 3 in place of PT and FHA were used as an antigen for sensitization. The antigen-sensitized solid phase thus obtained were used in assay. The results are shown in Table 3.

It is clear from Table 3 that in either of antigens used for sensitization as listed in Table 3, the antigen-sensitized solid phase according to the present invention are superior in assay sensitivity to non-GA treated ones.

Either of antigens used for sensitization as listed in Example 3 was prepared by the known methods as listed below.

① Tetanus toxoid: prepared from a culture supernatant of H47 A strain according to the method of Pillemer, et al. [J. Biol. Chem. 173 , 427 (1948)]

② Diphtheria toxoid: prepared from a culture supernatant of PW-No.8, Toronto strain according to the method of Yoneda, et al. [Tanpakudokuso (Proteinous Toxin), Vol. I, 152-154 (1972), Koudan-sha, Japan]

③ Rubella virus: prepared from a cell culture of PRK infected with TO-336 strain by the sucrose density gradient centrifugation. [Virus Jitsukengaku, Souron, Ed by Kokuritsu Yoboueisei Kenkyu sho Gakuyuu-kai, Maruzen, Japan]

④ Mumps virus: prepared from an amniotic fluid of embryonating egg, infected with Enders strain by the method of Sugiura, et al. [Rinshou To Virus, 12(1) , 81-86 (1984)].

⑤ 60K OMP: prepared from a culture fluid of Bordetella pertussis Tohama Phase I strain by the method of Brenan, et al. [Infect. Immun. 56 , 3189-3195 (1988)].

⑥ Fimbriae: prepared from a culture fluid of Bordetella pertussis Tohama Phase I strain by the method of Irons, et al. [Develop. Biol. Standard, 61 , 153-163 (1985)].

Table 3

①Antibody Titer of Anti-Tetanus

| Sensitizing Antigen | Final GA concen- tration (%) | Standard a-Tetanus (EU/ml) | | | | | |
|---|---|---|---|---|---|---|---|
| | | 9.06 | 2.27 | 0.566 | 0.142 | 0.036 | 0 |
| Tetanus Toxoid | 12.5 | 1063 | 552 | 214 | 73 | 23 | 4 |
| | 2.5 | 1074 | 514 | 252 | 78 | 18 | 5 |
| | 0.5 | 937 | 510 | 186 | 53 | 15 | 4 |
| | 0 | 477 | 185 | 63 | 16 | 6 | 3 |

②Antibody Titer of Anti-Diphtheria

| Sensitizing Antigen | Final GA concen- tration (%) | Standard a-Diphtheria (EU/ml) | | | | | |
|---|---|---|---|---|---|---|---|
| | | 1.02 | 0.255 | 0.064 | 0.016 | 0.004 | 0 |
| Diphtheria Toxoid | 12.5 | 981 | 445 | 166 | 44 | 14 | 2 |
| | 2.5 | 929 | 410 | 123 | 39 | 13 | 1 |
| | 0.5 | 869 | 421 | 128 | 31 | 10 | 2 |
| | 0 | 785 | 343 | 115 | 33 | 7 | 1 |

③Antibody Titer of Anti-Rubella Virus

| Sensitizing Antigen | Final GA concen- tration (%) | a-Rubella (Dilution) | | | | | |
|---|---|---|---|---|---|---|---|
| | | $1^x$ | $2^x$ | $4^x$ | $8^x$ | $16^x$ | $32^x$ |
| Rubella Virus | 12.5 | 666 | 404 | 164 | 52 | 19 | 8 |
| | 2.5 | 783 | 473 | 204 | 64 | 22 | 8 |
| | 0.5 | 716 | 436 | 180 | 54 | 19 | 7 |
| | 0 | 738 | 371 | 142 | 41 | 17 | 7 |

Data given in terms of absorbance (x 1000)

④Antibody Titer of Anti-Mumps Virus

| Sensitizing Antigen | Final GA concentration (%) | a-Mumps (Dilution) | | | | | |
|---|---|---|---|---|---|---|---|
| | | $1^x$ | $2^x$ | $4^x$ | $8^x$ | $16^x$ | $32^x$ |
| Mumps Virus | 12.5 | 1035 | 708 | 268 | 86 | 25 | 4 |
| | 2.5 | 1065 | 703 | 270 | 80 | 22 | 3 |
| | 0.5 | 1016 | 677 | 262 | 73 | 24 | 4 |
| | 0 | 866 | 619 | 230 | 62 | 19 | 3 |

⑤Antibody Titer of Anti-Pertussis 69K OMP

| Sensitizing Antigen | Final GA concentration (%) | a-60K OMP (EU/ml) | | | | | |
|---|---|---|---|---|---|---|---|
| | | 300 | 75 | 18.8 | 4.7 | 1.2 | 0 |
| 69K OMP | 12.5 | 1183 | 542 | 220 | 87 | 28 | 8 |
| | 2.5 | 1257 | 772 | 261 | 92 | 33 | 12 |
| | 0.5 | 1113 | 664 | 238 | 95 | 30 | 10 |
| | 0 | 886 | 446 | 161 | 61 | 14 | 1 |

⑥Antibody Titer of Anti-Pertussis Fimbriae

| Sensitizing Antigen | Final GA concentration (%) | a-Fimbriae (EU/ml) | | | | | |
|---|---|---|---|---|---|---|---|
| | | 64 | 16 | 4 | 1 | 0.25 | 0 |
| Fimbriae | 12.5 | 1205 | 585 | 209 | 57 | 15 | 0 |
| | 2.5 | 1221 | 597 | 198 | 58 | 15 | 1 |
| | 0.5 | 1085 | 589 | 181 | 51 | 13 | 0 |
| | 0 | 1070 | 508 | 161 | 44 | 10 | 1 |

Data given in terms of absorbance (x 1000)

Example 4

Polystyrene beads were treated according to the same manner as described in Example 2 except that various aldehydes listed in Table 4 in place of 2.5% (v/v) aqueous solution of acetaldehyde (AA) were used. The antigen-sensitized solid phases thus obtained were used in the assay. The results are shown in Table 4.

It is clear from Table 4 that the antigensensitized solid phases which have been pretreated with various aldehydes listed in Table 4 are superior in assay sensitivity to those which have not yet been so pretreated.

Table 4

| Sensitizing antigen | Aldehyde | Final concentration (%) | Standard a-FHA (EU/ml) | | | | | |
|---|---|---|---|---|---|---|---|---|
| | | | 200 | 50 | 12.5 | 3.12 | 0.78 | 0 |
| FHA | No treatment | | 760 | 409 | 149 | 60 | 30 | 23 |
| | Acetaldehyde | 12.5 | 926 | 461 | 182 | 77 | 56 | 24 |
| | | 2.5 | 897 | 460 | 179 | 70 | 30 | 20 |
| | | 0.5 | 1044 | 522 | 189 | 97 | 50 | 27 |
| | Glyoxal | 12.5 | 805 | 406 | 161 | 56 | 34 | 20 |
| | | 2.5 | 928 | 432 | 124 | 50 | 29 | 18 |
| | | 0.5 | 924 | 474 | 163 | 71 | 41 | 28 |
| | Pyruvic aldehyde | 12.5 | 1018 | 520 | 172 | 65 | 30 | 14 |
| | | 2.5 | 944 | 458 | 155 | 67 | 25 | 17 |
| | | 0.5 | 978 | 466 | 158 | 64 | 32 | 22 |
| | Formaldehyde | 12.5 | 1111 | 609 | 236 | 94 | 45 | 29 |
| | | 2.5 | 1116 | 632 | 217 | 85 | 36 | 22 |
| | | 0.5 | 798 | 406 | 144 | 46 | 21 | 20 |
| Data given in terms of absorbance (x 1000) | | | | | | | | |

## Claims

1. A synthetic resin bead sensitized with an antigen, which is treated with an aldehyde prior to sensitization.

2. The bead according to Claim 1, wherein the aldehyde is glutaraldehyde.

3. The bead according to Claim 1, wherein the bead is treated with from about 0.8 to about 2.5 % (v/v) of the aldehyde at from about 20°C to about 3°C for about 1 to 2 hours.

4. The bead according to Claim 1, wherein the antigen is selected from active components of the protective antigen.

5. The bead according to Claim 1, wherein the antigen is derived from pathogenic micro-organisms.

6. The bead according to Claim 5, wherein the pathogenic micro-organism is selected from the group consisting of Bordetella pertussis , Clostridium tetani , Corynebacterium diphtheriae , measles virus, rubella virus, mumps virus, and Japanese encephalitis virus.

7. The bead according to Claim 1, wherein the antigen is derived from Bordetella pertussis .

8. The bead according to Claim 7, wherein the antigen derived from Bordetella pertussis is selected from the group consisting of filamentous hemagglutinin and pertussis toxin.

9. The bead according to Claim 1, wherein the bead is a spheroidal, synthetic resin and has a diameter of from about 2 to about 9 mm.

10. The bead according to Claim 9, wherein the bead is a spherical, synthetic resin and has a diameter of from about 3 to about 8 mm.

11. The bead according to Claim 1, wherein the synthetic resin bead has a surface resulting from abrasion.

12. The bead according to Claim 1, wherein the synthetic resin is selected from polystyrene resins.

13. The bead according to Claim 1, wherein the synthetic resin is selected from synthetic resins which comprise mainly polystyrene.

14. A method for producing the bead according to any of Claims 1-13, which comprises sensitizing a synthetic resin bead with an antigen after the treatment with a compound selected from aldehydes.

15. An enzyme immunoassay for determining an antibody against an antigen in a sample, which comprises using the bead according to any of Claims 1-13 as a solid phase.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 140 489 (WAKO PURE CHEMICAL INDUSTRIES, LTD)<br>* Page 5, lines 11-22; page 6, line 21 - page 7, line 27 * | 1,2,4,5,12-15 | G 01 N 33/546<br>G 01 N 33/569 |
| X | US-A-4 224 198 (A. REMBAUM et al.)<br>* Column 1, lines 45-47; column 2, line 54 - column 3, line 2; column 6, line 59 - column 7, line 31 * | 1,2,4,12-15 | |
| X | EP-A-0 026 007 (TETRA CONSULTANTS INC.)<br>* Page 1, line 25 - page 4, line 1; page 7, line 25 - page 8, line 16 * | 1,2,4,12-14 | |
| Y | | 5-8 | |
| Y | EP-A-0 001 224 (F. HOFFMAN-LA ROCHE & CO.)<br>* Page 2, line 25 - page 3, line 30; page 5, lines 12-17; page 6, line 1 * | 5-8 | |
| Y | BIOLOGICAL ABSTRACTS, vol. 87, no. 11, 1989, page 589, abstract no. 116400, Philadelphia, PA, US; S.A. HALPERIN et al.: "Evaluation of culture, immunofluorescense, and serology for the diagnosis of pertussis", & J. CLIN. MICROBIOL. 27(4): 752-757. 1989<br>* Abstract * | 5-8 | TECHNICAL FIELDS SEARCHED (Int. Cl.5)<br><br>G 01 N |
| X,P | EP-A-0 341 498 (E.I. DU PONT DE NEMOURS & CO.)<br>* Page 3, lines 40-46; page 4, lines 5-21; page 5, lines 20-35 *<br>-/- | 1-4,12-15 | |

The present search report has been drawn up for all claims

| Place of search | Date of completion of the search | Examiner |
|---|---|---|
| THE HAGUE | 20-12-1990 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 5) |
|---|---|---|---|
| A | US-A-4 001 583 (M.J. BARRETT) * Abstract; column 2, line 12 - column 3, line 7 * ----- | 1-4 | |
| | | | TECHNICAL FIELDS SEARCHED (Int. Cl.5) |

The present search report has been drawn up for all claims

| Place of search | Date of completion of the search | Examiner |
|---|---|---|
| THE HAGUE | 20-12-1990 | HITCHEN C.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)